# EUROPEAN PATENT APPLICATION

(11) **EP 4 524 164 A1**
(43) Date of publication of application: **19.03.2025**
(21) Application number: 23803056.3
(22) Date of filing: 15.05.2023
(51) Int. Cl.: C07K 19/00, C12N 15/85, A61K 38/19, A61K 38/20, A61K 47/65, A61K 47/68, A61P 35/00

(54) **NOVEL FUSION PROTEIN AND USE THEREOF**

(30) Priority: 13.05.2022 CN 202210521837
(71) Applicant: Nanjing Jsiama Biopharmaceuticals Ltd., Jiangbei New District Nanjing, Jiangsu 210043 (CN)
(72) Inventor: LIU, Liming, Nanjing, Jiangsu 210043 (CN); HAN, Zhen, Nanjing, Jiangsu 210043 (CN); KANG, Ping, Nanjing, Jiangsu 210043 (CN)
(74) Representative: Eisenführ Speiser
(86) International application number: PCT/CN2023/094205
(87) International publication number: WO 2023/217288

(57) **Abstract**

Provided are modifications of human interleukin-2 (IL-2) and interferon α (IFNα), a fusion protein formed by modified IL-2, IFNα or both of IL-2/IFNα and an Fc, and design, preparation and use of the fusion protein. By changing the binding capacity of IFNα to a receptor thereof by means of gene mutation, a superior fusion protein is acquired, which may overcome the defects of weak specificity and serious adverse effects of existing drugs.

## Description

### CROSS-REFERENCE

The instant application claims priority to Chinese Patent Application No. 202210521837.5, filed on May 13, 2022, which is incorporated herein by reference in its entirety.

### SEQUENCE LISTING

The instant application contains a sequence listing, and its entire content is incorporated herein by reference.

### FIELD

The present invention belongs to the field of biomedical technology, and specifically relates to the design, preparation and use of novel IL-2 fusion proteins with IFNα and Fc.

### BACKGROUND

IL-2, also known as T-cell growth factor, has a gene located on chromosome 4, containing a sequence of 7 kb in total. IL-12 protein consists of 133 amino acids with a molecular weight of approximately 15 kD. IL-2 acts through IL-2R, which includes three subunits, IL-2Rα (i.e., CD25), IL-2Rβ (i.e., CD122), and IL-2Rγ (i.e., CD132). The three subunits can form three receptor forms: the high-binding affinity receptor comprising all of the three subunits IL-2Rα/β/γ, the medium-binding affinity receptor comprising the two subunits IL-2Rβ/γ, and the low-binding affinity receptor being IL-2Rα. Among them, IL-2Rβ and IL-2Rγ are necessary for IL-2 to activate downstream signaling pathways. When IL-2 binds to both IL-2Rβ and IL-2Rγ, the two receptor subunits form a heterodimer that in turn phosphorylates intracellular STAT5, subsequently the phosphorylated STAT5 enters the cell nucleus leading to the transcription and expression of corresponding genes. Whereas IL-2Rα is not necessary for signaling, it can promote the binding of IL-2 to IL-2Rβ and IL-2Rγ. IL-2Rγ is expressed in all immune cells; IL-2Rβ is expressed in CD8+T cells, NK cells, and regulatory T cells, and its expression level will be elevated after T cells are activated; IL-2Rα is continuously, highly expressed in regulatory T cells, and transiently expressed in the activated CD8+T cells which is followed by downregulation of the expression level[1,2]. IL-2 is the second immunotherapy approved to treat metastatic melanoma (1988) and renal cell carcinoma (1992); IFNα is the first immunotherapy approved (1986) for the treatment of hairy cell leukemia, and later approved for the treatment of metastatic melanoma, renal cell carcinoma, non-Hodgkin's lymphoma and Kaposi's sarcoma.

IL-2 drugs have a relatively short half-life, consequently, IL-2 drugs that have been approved to treat tumors require high doses to be effective. However, high doses of IL-2 will result in obvious toxic side effects, preventing their widespread usage. Their selectivity for NK cells and CD8+T cells expressing IL-2Rβ and IL-2Rγ is relatively low, therefore cannot fully exert the ability of NK cells and CD8+T cells to kill tumors[3,4].

On the other hand, IFNα is a cytokine produced by immune cells in the body, which is a group of low-molecular-weight glycoproteins with similar structures and functions produced by immune cells through an antiviral response when the body is infected by a virus. Interferon plays a very important role in the body's immune system[5,6]. Recombinant human interferon α has been approved for the treatment of viral infections and tumors. Since interferon α receptors are widely distributed and expressed in many normal cells, it is prone to causing a significant toxic side effect.

Therefore, there is a need in the art to generate new cytokine constructs to provide more effective therapeutic effects while minimizing side effects.

### SUMMARY

In order to address the above issues, the present invention discloses the design, preparation and use of novel IL-2 fusion proteins with IFNα and Fc, which changes the binding ability of IL-2 to its receptor(s) and IFNα to its receptor(s) by means of gene mutation to overcome the shortcomings of weak specificity, short half-life and serious side effects of existing drugs.

In some aspects, the present disclosure provides a fusion protein comprising an IL-2 moiety and an Fc moiety, wherein the IL-2 moiety comprises an amino acid sequence with one or more mutations compared to wild-type IL-2 protein, and the amino acid sequence has at least 90% identity to SEQ ID NO: 2.

In some embodiments, the mutations include one or more substitutions selected from the following with reference to amino acid positions in SEQ ID NO: 1: R38A, L80F, R81D, L85V, I86V and I91F.

In some embodiments, the Fc moiety comprises a human IgG Fc, such as human IgG1 Fc, IgG2 Fc, IgG3 Fc, IgG4 Fc or variants thereof. For example, the Fc variant comprises one or more mutations selected from the following: L234A and L235A mutations, and M252Y, S254T and T256E mutations.

In some embodiments, the fusion protein further comprises an IFNα moiety. The IFNα moiety may comprise an amino acid sequence with at least 90% identity to SEQ ID NO: 6 or 5. In some embodiments, the IFNα moiety includes one or more substitutions selected from the following with reference to amino acid positions in SEQ ID NO: 5: R144A and R149A.

In some embodiments, the fusion protein comprises:
(a) an IL-2 moiety operably linked to an Fc moiety;
(b) an IL-2 moiety operably linked to an Fc moiety, and the Fc moiety operably linked to an IFNα moiety;
(c) an IL-2 moiety operably linked to an IFNα moiety, and the IFNα moiety operably linked to an Fc moiety; or
(d) an IFNα moiety operably linked to an IL-2 moiety, and the IL-2 moiety operably linked to an Fc moiety.

In some further embodiments, the fusion protein comprises:
(a) an IL-2 moiety operably linked to an Fc moiety;
(b) an IL-2 moiety operably linked to an Fc moiety, and the Fc moiety operably linked to an IFNα moiety;
(c) an Fc moiety operably linked to an IL-2 moiety; or
(d) an IFNα moiety operably linked to an Fc moiety, and the Fc moiety operably linked to an IL-2 moiety.

In some embodiments, the operable linkage is either a direct linkage or a linking via a peptide linker. Optionally, the peptide linker is a GS series linker, such as (GS)n, where n=1-5. In some embodiments, the linker is as set forth in SEQ ID NO: 11.

In some embodiments, the fusion protein comprises an amino acid sequence as set forth in SEQ ID NO: 3, 4, 9 or 10.

In some aspects, the present disclosure provides a fusion protein comprising an IFNα moiety and an Fc moiety, wherein the IFNα moiety comprises an amino acid sequence with one or more mutations compared to wild-type IFNα protein, and the amino acid sequence is an amino acid sequence with at least 90% identity to SEQ ID NO: 6.

In some embodiments, the IFNα moiety includes one or more substitutions selected from the following with reference to amino acid positions in SEQ ID NO: 5: R144A and R149A.

In some embodiments, the Fc moiety comprises a human IgG Fc, such as human IgG1 Fc, IgG2 Fc, IgG3 Fc, IgG4 Fc or variants thereof. For example, the Fc variant comprises one or more mutations selected from the following: L234A and L235A mutations, and M252Y, S254T and T256E mutations.

In some embodiments, the fusion protein comprises, from N-terminal to C-terminal:
(a) an IFNα moiety operably linked to an Fc moiety; or
(b) the Fc moiety operably linked to an IFNα moiety.

In some embodiments, the fusion protein comprises an amino acid sequence as set forth in SEQ ID NO: 7 or 8.

In some aspects, the present disclosure provides a nucleic acid molecule comprising a nucleic acid sequence encoding the fusion protein disclosed herein.

In some aspects, the present disclosure provides a vector comprising the nucleic acid molecule disclosed herein.

In some aspects, the present disclosure provides a host cell comprising the nucleic acid molecule or the vector as disclosed herein.

In some aspects, the present disclosure provides a pharmaceutical composition comprising the fusion protein or the nucleic acid molecule encoding the same as disclosed herein, and a pharmaceutically acceptable carrier.

In some aspects, the present disclosure provides a method for producing the fusion protein disclosed herein, comprising the following steps:
- expressing the fusion protein in a host cell comprising a vector encoding the fusion protein; and
- isolating the fusion protein from the host cell culture.

In some aspects, the present disclosure provides a method for modulating an immune response in a subject, comprising administering to the subject the fusion protein or pharmaceutical composition disclosed herein.

In some aspects, the present disclosure provides a method for treating or preventing a cancer or an infectious disease in a subject, comprising administering to the subject an effective amount of the fusion protein or pharmaceutical composition disclosed herein.

In some embodiments, the cancer is selected from the group consisting of breast cancer, gastric cancer, melanoma, lymphoma, lung cancer, colon cancer, ovarian cancer, bladder cancer, renal cell carcinoma, liver cancer, prostate cancer, pancreatic cancer and leukemia.

In some aspects, the present disclosure provides an use of the fusion protein disclosed herein in the manufacture of a medicament for the prevention, treatment and/or management of a cancer or an infectious disease.

In some aspects, the present disclosure provides the fusion protein disclosed herein for use in the treatment or prevention of a cancer or an infectious disease.

In some aspects, the present disclosure provides a kit comprising a container containing the fusion protein or pharmaceutical composition disclosed herein.

The invention also relates to the following embodiments:
1. A novel fusion protein of IL-2 with IFNα and Fc, characterized by comprising: a fusion protein of IL-2 and IFNα dual factors and Fc, a fusion protein of IL-2 and Fc, a fusion protein of IFNα and Fc;
   the fusion protein of IL-2 and IFNα dual factors and Fc: a mutant IL-2 and a mutant IFNα are fused to the N-terminal or C-terminal of Fc, respectively, to obtain a polypeptide chain IL-2-Fc-IFNα or IFNα-Fc-IL-2;
   the fusion protein of IL-2 and Fc: a mutant IL-2 is fused to the N-terminal or C-terminal of Fc to obtain a polypeptide chain Fc-IL-2 or IL-2-Fc;
   the fusion protein of IFNα and Fc: a mutant IFNα is fused to the N-terminal or C-terminal of Fc to obtain a polypeptide chain Fc-IFNα or IFNα-Fc.
2. The novel fusion protein of IL-2 with IFNα and Fc according to embodiment 1, characterized in that, in the fusion protein of IL-2 and IFNα dual factors and Fc, the Fc fragment comprises a hinge region of the heavy chain constant region, a second domain CH2 of the heavy chain constant region, and a third domain CH3 of the heavy chain constant region.
3. The novel fusion protein of IL-2 with IFNα and Fc according to embodiment 1, characterized in that, in the fusion protein of IL-2 and Fc, the Fc fragment comprises a hinge region of the heavy chain constant region, a second domain CH2 of the heavy chain constant region, and a third domain CH3 of the heavy chain constant region.
4. The novel fusion protein of IL-2 with IFNα and Fc according to embodiment 1, characterized in that, in the fusion protein of IFNα and Fc, the Fc fragment comprises a hinge region of the heavy chain constant region, a second domain CH2 of the heavy chain constant region, and a third domain CH3 of the heavy chain constant region.
5. The novel fusion protein of IL-2 with IFNα and Fc according to any one of embodiments 1-2, characterized in that, in the fusion protein of IL-2 and IFNα dual factors and Fc, L234A and L235A mutations, along with M252Y, S254T and T256E mutations, are further introduced into the Fc fragment.
6. The novel fusion protein of IL-2 with IFNα and Fc according to any one of embodiment 1 or 3, characterized in that, in the fusion protein of IL-2 and Fc, L234A and L235A mutations, along with M252Y, S254T and T256E mutations, are further introduced into the Fc fragment.
7. The novel fusion protein of IL-2 with IFNα and Fc according to any one of embodiment 1 or 4, characterized in that, in the fusion protein of IFNα and Fc, L234A and L235A mutations, along with M252Y, S254T and T256E mutations, are further introduced into the Fc fragment.
8. The novel fusion protein of IL-2 with IFNα and Fc according to embodiment 1, characterized in that, in the fusion protein of IL-2 and IFNα dual factors and Fc, the IL-2 mutant is obtained by introducing mutations on the basis of wild-type IL-2 through molecular biological means, and the introduced mutations include, but are not limited to, R38A, L80F, R81D, L85V, I86V and I91F.
9. The novel fusion protein of IL-2 with IFNα and Fc according to embodiment 3, characterized in that, in the fusion protein of IL-2 and Fc, the IL-2 mutant is obtained by introducing mutations on the basis of wild-type IL-2 through molecular biological means, and the introduced mutations include, but are not limited to, R38A, L80F, R81D, L85V, I86V and I91F.
10. The novel fusion protein of IL-2 with IFNα and Fc according to embodiment 1, characterized in that, in the fusion protein of IL-2 and IFNα dual factors and Fc, the IFNα derivative is obtained by introducing mutations on the basis of wild-type IFNα through molecular biological means, and the introduced mutations include, but are not limited to, R144A or R149A.
11. The novel fusion protein of IL-2 with IFNα and Fc according to embodiment 4, characterized in that, in the fusion protein of IFNα and Fc, the IFNα derivative is obtained by introducing mutations on the basis of wild-type IFNα through molecular biological means, and the introduced mutations include, but are not limited to, R144A or R149A.
12. A method for preparing the novel fusion protein of IL-2 with IFNα and Fc as described in any one of embodiments 1-2, characterized in that, the method for preparing the fusion protein of IL-2 and IFNα dual factors and Fc comprises the following steps:
   (1) linking an IL-2 mutant to the N-terminal or C-terminal of a Fc fragment through a flexible linker, and linking an IFNα derivative to the C-terminal or N-terminal of the Fc fragment through a flexible linker, to obtain a polypeptide chain IL-2-Fc-IFNα or IFNα-Fc-IL-2; introducing mutations L234A, L235A, M252Y, S254T, and T256E into the Fc fragment;
   (2) cloning the DNA fragment obtained in step (1) into a pcDNA series vector or other vectors used in mammalian cell expression systems to obtain a recombinant vector;
   (3) transfecting the recombinant vector obtained in step (2) into mammalian cells to express the fusion protein, and then purifying the resulting product to obtain the fusion protein of IL-2 and IFNα dual-factor and Fc.
13. A method for preparing the novel fusion protein of IL-2 with IFNα and Fc as described in any one of embodiment 1 or 3, characterized in that the method for preparing the fusion protein of IL-2 and Fc comprises the following steps:
   (1) linking an IL-2 mutant to the N-terminal or C-terminal of a Fc fragment through a flexible linker to obtain a polypeptide chain IL-2-Fc or Fc-IL-2; introducing mutations L234A, L235A, M252Y, S254T, and T256E into the Fc fragment;
   (2) cloning the DNA fragment obtained in step (1) into a pcDNA series vector or other vectors used in mammalian cell expression systems to obtain a recombinant vector;
   (3) transfecting the recombinant vector obtained in step (2) into mammalian cells to express the fusion protein, and then purifying the resulting product to obtain the fusion protein of IL-2 and Fc.
14. A method for preparing the novel fusion protein of IL-2 with IFNα and Fc as described in any one of embodiment 1 or 4, characterized in that the method for preparing the fusion protein of IFNα and Fc comprises the following steps:
   (1) linking an IFNα derivative to the C-terminal or N-terminal of a Fc fragment through a flexible linker to obtain a polypeptide chain Fc-IFNα or IFNα-Fc; and introducing mutations L234A, L235A, M252Y, S254T, and T256E into the Fc fragment;
   (2) cloning the DNA fragment obtained in step (1) into a pcDNA series vector or other vectors used in mammalian cell expression systems to obtain a recombinant vector;
   (3) transfecting the recombinant vector obtained in step (2) into mammalian cells to express the fusion protein, and then purifying the resulting product to obtain the fusion protein of IFNα and Fc.
15. The method for preparing the novel IL-2 fusion protein with IFNα and Fc according to any one of embodiments 12-14, characterized in that in step (3), the mammalian cells include HEK293 cells, CHO cells or any cells derived therefrom.
16. The method for preparing the novel IL-2 fusion protein with IFNα and Fc according to any one of embodiments 12-14, characterized in that the introduction of mutations into the Fc fragment in step (1) is specified as: the introduction of L234A and L235A mutations into the Fc fragment, along with the introduction of the M252Y, S254T and T256E mutations into the Fc fragment.
17. Use of the novel fusion protein of IL-2 with IFNα and Fc described in any one of embodiments 1-11 in the manufacture of a broad-spectrum antitumor medicament, comprising a monotherapy for the treatment of the approved tumor therapeutic indications of IL-2 and IFNα, as well as a broad-spectrum tumor therapy in combination with other tumor therapeutic methods.
18. Use of the novel fusion protein of IL-2 with IFNα and Fc prepared by the method described in any one of embodiments 12-16 in the manufacture of a Fc fusion protein medicament, comprising a monotherapy for the treatment of the approved tumor therapeutic indications of IL-2 and IFN-a, as well as a broad-spectrum tumor therapy in combination with other tumor therapeutic methods.

The foregoing is an overview and thus simplifications, generalizations, and omissions of details are included if necessary; consequently, those skilled in the art will appreciate that the overview is illustrative only and is not intended to be in any way limiting. Other aspects, features and advantages of the methods, compositions and uses and/or other subject matters described herein will become apparent in the teachings set forth herein.

### BRIEF DESCRIPTION OF THE DRAWINGS

Figure 1A exemplifies structural diagrams of various Fc fusion proteins encompassed by the present invention. The direction from top to bottom of each panel indicates the order from N-terminal to C-terminal, and the trapezoid represents the hinge region.
Figures 1B-1C exemplify an SDS PAGE expression profile and HPLC analysis graph of the Fc fusion protein of the present invention, respectively.
Figure 2 exemplifies a graph for binding capacity detection of the Fc fusion protein of the present invention to the receptor IL-2Rα protein.
Figure 3 exemplifies a graph for binding capacity detection of the Fc fusion protein of the present invention to the receptor IL-2Rβ protein.
Figure 4 exemplifies a graph for binding capacity detection of the Fc fusion protein of the present invention to the receptor IFNαR2 protein.
Figure 5 exemplifies a graph for binding capacity detection of the Fc fusion protein of the present invention to receptor IL-2Rα proteins across different species.
Figure 6 exemplifies a graph for binding capacity detection of the Fc fusion protein of the present invention to receptor IL-2Rβ proteins across different species.
Figure 7 exemplifies a graph for binding capacity detection of the Fc fusion protein of the present invention to receptor IFNαR2 proteins across different species.
Figure 8A and Figure 8B exemplify graphs for binding capacity results of the Fc fusion protein of the present invention to the FcRn protein as detected by ELISA and Fortebio, respectively.
Figure 9 exemplifies charts for the distribution detection of specific cell subsets of PBMC stimulated by the Fc fusion protein of the present invention.
Figure 10 exemplifies the detection of the ability of the Fc fusion protein of the present invention to stimulate the proliferation of CTLL-2 mouse T lymphocytes.
Figures 11A to 11D exemplify the detection of the in vitro direct killing ability of the Fc fusion protein of the present invention against tumor cells (A: NCI-N87; B: MDA-MB-231; C: A375; D: Burkitt's lymphoma cells).
Figures 12A to 12E exemplify the detection of a comprehensive killing ability of the Fc fusion protein of the present invention against tumor cells *in vitro*(A: NCI-N87; B: MDA-MB-231; C: A375; D and E: Burkitt's lymphoma cells).
Figure 13 exemplifies the pharmacodynamic activity of the Fc fusion protein of the present invention in an ExVivo organoid system.

### DETAILED DESCRIPTION

Unless defined otherwise, all technical and scientific terms used herein have the same meaning as commonly understood by one of ordinary skill in the art to which the present disclosure belongs. All patents, patent applications, and other publications cited herein are incorporated by reference in their entirety. To the extent that definitions set forth herein conflict with those set forth in patents, patent applications, and other publications incorporated herein by reference, the definitions set forth herein shall control.

As used herein, the term "IL-2" refers to interleukin-2 and is intended to include any form of IL-2, e.g., 1) a native, unprocessed IL-2 molecule, full-length IL-2 protein, or naturally occurring IL-2 variant; 2) any form of IL-2 produced by intracellular processing; or 3) a full-length or modified form. In the present disclosure, the term "IL-2" or "IL-2 domain" includes wild-type IL-2 and an IL-2 variant.

As used herein, the term "IFNα" refers to interferon α and is intended to include any form of IFNα, e.g., 1) a native, unprocessed IFNα molecule, full-length IFNα protein, or naturally occurring IFNα variant; 2) any form of IFNα produced by intracellular processing; or 3) a full-length or modified form. In the present disclosure, the term "IFNα" or "IFNα domain" includes wild-type IFNα and an IFNα variant.

The term "variant", with respect to a polypeptide or protein, refers to a biologically active polypeptide that includes one or more amino acid mutations to the native protein sequence. Optionally, the one or more amino acid mutations include amino acid substitutions and/or insertions at certain positions in the amino acid sequence. Preferably, the variant shares at least about 80% amino acid sequence identity with the corresponding native sequence polypeptide. Such variants include, for example, a polypeptide in which one or more amino acid (naturally occurring amino acid and/or non-naturally occurring amino acid) residues have been added to the N-terminal and/or C-terminal of the polypeptide. Variants for use in the present disclosure can be prepared by a variety of methods well-known in the art, such as site-directed mutagenesis on nucleotides in the DNA encoding the native protein or phage display techniques, to generate DNAs encoding the variants followed by expression of the DNAs in recombinant cell cultures. In certain embodiments disclosed herein, the IL-2 variant includes one or more substitutions compared to wild-type IL-2 protein. In certain embodiments disclosed herein, the IFNα variant includes one or more substitutions compared to wild-type IFNα protein.

The term "Fc" as used herein has the same meaning as used with respect to an antibody and refers to that portion of the antibody comprising the second (CH2) and third (CH3) constant regions of a first heavy chain bound to the second and third constant regions of a second heavy chain through disulfide bonds. The Fc region may also include part or all of the hinge region. The Fc region of an antibody is responsible for various effector functions such as ADCC and CDC, but does not have an antigen-binding function. In this disclosure, the term "Fc" includes a wild-type Fc and an Fc variant.

As used herein, the term "operably linked" refers to a juxtaposition (with or without a spacer or linker or insertion sequence) of two or more biological sequences of interest such that they are in a relationship permitting them to function in their intended manner. When used with respect to polypeptides, it means that the polypeptide sequences are linked in such a way that permits the linked product to have the intended biological function. For example, a fusion protein may be operably linked to a constant region of immunoglobulin so as to provide for a stable product with ligand-binding activity. For another example, a fusion protein may be operably linked to a constant region of immunoglobulin through an insertion sequence therebetween, and such insertion sequences may be spacers or may contain longer sequences.

As used herein, the term "fusion" or "fused" when used with respect to amino acid sequences (e.g., peptide, polypeptide or protein) refers to a combination of two or more amino acid sequences, for example by chemical bonding or recombinant means, into a single amino acid sequence which does not exist naturally. A fusion amino acid sequence may be produced by genetic recombination of two encoding polynucleotide sequences, and can be expressed by a method of introducing a construct containing the recombinant polynucleotide into a host cell.

The term "fusion protein" as used herein refers to a polypeptide having two (or more) moieties operably linked together, where each of the moieties is a polypeptide having a different property. The property may be a biological property, such as activity in vitro or in vivo. The property can also be a simple chemical or physical property, such as binding to a target antigen, catalysis of a reaction, etc. The two moieties can be linked directly by a single peptide bond or linked through a peptide linker containing one or more amino acid residues. Generally, the two moieties and the linker will be in the reading frame with each other. In certain embodiments, the fusion protein is a fusion protein of an IL-2 moiety, an Fc moiety and an IFNα moiety. In certain embodiments, the fusion protein is a fusion protein of an IL-2 moiety and an Fc moiety. In certain embodiments, the fusion protein is a fusion protein of an Fc moiety and an IFNα moiety.

As used herein, the term "vector" refers to a nucleic acid vehicle which can have a polynucleotide inserted therein. When the vector allows for the expression of the protein encoded by the polynucleotide inserted therein, the vector is called an expression vector. The vector can have the carried genetic material elements expressed in a host cell by transformation, transduction, or transfection into the host cell. Vectors are well-known to those skilled in the art, including, but not limited to plasmids, phagemids, cosmids, artificial chromosomes such as yeast artificial chromosome (YAC), bacterial artificial chromosome (BAC) or P1-derived artificial chromosome (PAC); bacteriophages, such as λ bacteriophage or M13 bacteriophage and animal viruses, etc. The animal viruses that may be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise multiple elements for controlling expression, including, but not limited to, a promoter sequence, a transcription initiation sequence, an enhancer sequence, a selection element and a reporter gene. In addition, a vector may comprise an origin of replication.

As used herein, the term "host cell" refers to a cellular system which can be engineered to generate proteins, protein fragments, or peptides of interest. Host cells include, without limitation, cultured cells, e.g., mammalian cultured cells derived from rodents (rats, mice, guinea pigs, or hamsters) such as CHO, BHK, NSO, SP2/0, YB2/0; or human tissues or hybridoma cells, yeast cells, and insect cells, as well as cells comprised within a transgenic animal or cultured tissue. The term encompasses not only the particular subject cell but also the progeny of such a cell. Because certain modifications may occur in succeeding generations due to either mutations or environmental influences, such progenies may not be identical to the parent cell, but are still included within the scope of the term "host cell".

The term "identity", as used herein, refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAMJ. Applied Math. 48:1073.

The term "subject" includes any human or nonhuman animal, preferably humans.

As used herein, the term "cancer" refers to any tumor or malignant cell growth that triggers a medical condition, which can be a solid tumor or a non-solid tumor mediated by proliferation or metastasis.

The terms "treatment" and "treating", as used herein in the context of treating a condition, pertain generally to treatment and therapy, whether of a human or an animal, in which some desired therapeutic effects are achieved, for example, the inhibition of progression of the condition, and include a reduction in the rate of progress, a halt in the rate of progress, a regression of the condition, an amelioration of the condition, and a cure of the condition. Treatment as a prophylactic measure (i.e., prophylaxis, prevention) is also included. For cancer, "treatment" may refer to an inhibition or slowdown of tumor or malignant cell growth, proliferation or metastasis, or some combination thereof. For tumors, "treatment" includes a removal of all or part of the tumor, an inhibition or slowdown of tumor growth and metastasis, a prevention or delay of tumor progression, or some combination thereof.

As used herein, the term "effective amount" pertains to that amount of an active compound, or that amount of a material, composition or dosage form comprising an active compound, which is effective to produce certain desired therapeutic effects commensurate with a reasonable benefit/risk ratio, when administered in accordance with a desired treatment regimen.

### IL-2 variants and IFNα variants

In certain aspects, the present disclosure provides an IL-2 variant that comprises one or more modifications, such as insertions, deletions, and/or substitutions compared to a wild-type IL-2 protein, such as human wild-type IL-2 protein. The present disclosure further provides a fusion protein, comprising an IL-2 protein moiety fused to an Fc moiety. The IL-2 protein moiety may include a wild-type IL-2 protein, or include an IL-2 variant comprising one or more modifications (e.g., insertions and/or substitutions) compared to the wild-type IL-2 protein. In some embodiments, compared to a wild-type IL-2 protein, the IL-2 variant contains one or more substitutions selected from, but not limited to, the following: R38A, L80F, R81D, L85V, I86V and I91F. For example, the amino acid at position 38 is modified in the IL-2 variant compared to wild-type IL-2 protein. In some embodiments, the IL-2 variant further contains a conservative substitution(s) compared to wild-type IL-2 protein. In some embodiments, the IL-2 variant comprises or consists of the amino acid sequence as set forth in SEQ ID NO: 2. In some embodiments, the IL-2 variant comprises or consists of an amino acid sequence that is at least 85%, at least 90%, at least 95%, or at least 99% identical to SEQ ID NO: 2, e.g. at least 91%, 92%, 93%, 94%, 95%, 96%, 97%, 98%, or 99% identical to SEQ ID NO: 2.

In certain aspects, the present disclosure provides an IFNα variant that comprises one or more modifications, such as insertions, deletions, and/or substitutions compared to a wild-type IFNα protein, such as human wild-type IFNα protein. The present disclosure further provides a fusion protein, comprising an IFNα protein moiety fused to an Fc moiety. The IFNα protein moiety may include a wild-type IFNα protein, or include an IFNα variant comprising one or more modifications (e.g., insertions and/or substitutions) compared to the wild-type IFNα protein. In some embodiments, amino acids R144 and/or R149 in the IFNα variant are modified compared to wild-type IFNα protein. In some embodiments, the IFNα variant further contains a conservative substitution compared to a wild-type IFNα protein. In some embodiments, the IFNα variant comprises or consists of the amino acid sequence as set forth in SEQ ID NO: 6. In some embodiments, the IFNα variant comprises or consists of an amino acid sequence that is at least 85%, at least 90%, at least 95%, or at least 99% identical to SEQ ID NO: 6, e.g. at least 91%, 92%, 93%, 94%, 95%, 96%, 97%, 98%, or 99% identical to SEQ ID NO: 6.

### Fusion proteins of the present invention

In certain aspects, the present disclosure provides a fusion protein comprising an IL-2 moiety and an Fc moiety, a fusion protein comprising an IFNα moiety and an Fc moiety, and a fusion protein comprising an IL-2 moiety, an Fc moiety and an IFNα moiety. The fusion protein can be single chain or with multiple chains. In some embodiments, the fusion protein is a double chain homodimer or heterodimer. The respective IL-2 moiety, Fc moiety and IFNα moiety in each chain may be identical or different.

In some embodiments, the fusion protein of the present invention comprises, from N-terminal to C-terminal, an IL-2 moiety operably linked to an Fc moiety (also known as an IL-2-Fc fusion protein).

In some embodiments, the fusion protein of the present invention comprises, from N-terminal to C-terminal, an Fc moiety operably linked to an IL-2 moiety (also known as an Fc-IL-2 fusion protein).

In some embodiments, the fusion protein of the present invention comprises, from N-terminal to C-terminal, an IFNα moiety operably linked to an Fc moiety (also known as an IFNα-Fc fusion protein).

In some embodiments, the fusion protein of the present invention comprises, from N-terminal to C-terminal, an Fc moiety operably linked to an IFNα moiety (also known as an Fc-IFNα fusion protein).

In some embodiments, the fusion protein of the present invention comprises, from N-terminal to C-terminal, an IL-2 moiety operably linked to an Fc moiety, and the Fc moiety operably linked to an IFNα moiety (also known as an IL-2-Fc-IFNα fusion protein).

In some embodiments, the fusion protein of the present invention comprises, from N-terminal to C-terminal, an IFNα moiety operably linked to an Fc moiety, and the Fc moiety operably linked to an IL-2 moiety (also known as an IFNα-Fc-IL-2 fusion protein).

The IL-2 moiety comprised in the fusion protein may include a wild-type IL-2 protein or an IL-2 variant. The IFNα moiety comprised in the fusion protein may include a wild-type IFNα protein or an IFNα variant. The Fc moiety comprised in the fusion protein may include a wild-type Fc or an Fc variant.

In some embodiments, amino acid at position 38 in the IL-2 moiety comprised in the fusion protein is modified. The region adjacent to amino acid 38 has been found to play a key role in the increase in vascular permeability induced by IL-2, and thus modification of amino acid 38 can significantly reduce the vascular permeability.

In some embodiments, a modified interferon α moiety is comprised in the fusion protein, and it has a significantly reduced affinity for the interferon α receptor compared to a native interferon α. Such reduction can lead to a significant decrease in interferon α-induced toxic and side effects while retaining its antitumor activity. In some embodiments, amino acid R144 or R149 in the interferon α moiety comprised in the fusion protein is modified. These amino acids play a crucial role in the binding of interferon α to its receptor, and upon mutation, can cause a significant decrease in affinity without affecting its antitumor activity.

The operable linkage (or expressed as "-") is either a direct linkage of two moieties, or a linking via a linker such as a peptide linker. In some embodiments, the IL-2 moiety is operably linked to the Fc moiety via a peptide linker. In some embodiments, the IFNα moiety is operably linked to the Fc moiety via a peptide linker. The peptide linker can be any peptide linker commonly used in the art, such as a GS series linker, as set forth in SEQ ID NO: 11.

The fusion protein provided herein can improve the therapeutic effect, reduce the toxic and side effects, and expand the therapeutic safety window.

### Fusion protein comprising an Fc region

In some embodiments, the fusion protein provided herein further comprises a constant domain sequence of immunoglobulin, such as a constant domain sequence of human IgG, and more specifically, may comprise a hinge region and an Fc region, such as an Fc region sequence of IgG1, IgG2, IgG3, and IgG4. As known in the art, Fc refers to a portion of an antibody consisting of a second and a third constant regions of a first heavy chain of the antibody bound to a second and a third constant regions of a second heavy chain of the antibody via disulfide bonds, and optionally the Fc region further comprises all or part of a hinge region. The Fc region herein includes both a wild-type Fc region and variants thereof, with different mutations used for multiple purposes. The variant may contain one or more amino acid residue modifications, such as substitutions, in the Fc region.

In certain embodiments, the Fc region variant contains one or more amino acid substitutions that improve the pH-dependent binding to the neonatal Fc receptor (FcRn). Such a variant can have an extended pharmacokinetic half-life, as it binds to FcRn at an acidic pH which allows it to escape from degradation in the lysosome and then be translocated and released out of the cell. Methods of engineering an antibody molecule to improve the binding affinity to FcRn are well-known in the art, see, for example, Vaughn, D. et al., Structure, 6(1): 63-73, 1998; Kontermann, R. et al., Antibody Engineering, Volume 1, Chapter 27: Engineering of the Fc region for improved PK, Springer, 2010; Yeung, Y. et al., Cancer Research, 70: 3269-3277 (2010); and Hinton, P. et al., J. Immunology, 176: 346-356 (2006).

In certain embodiments, the fusion protein provided herein contains L234A/L235A substitutions to reduce the binding ability to the receptor FcgRIIIa. In certain embodiments, the fusion protein provided herein comprises one or more amino acid substitution(s) in the interface of the Fc region to facilitate and/or promote heterodimerization. These modifications comprise introduction of a protuberance into a first Fc polypeptide and a cavity into a second Fc polypeptide, wherein the protuberance can be positioned in the cavity so as to promote interaction of the first and second Fc polypeptides to form a heterodimer or a complex. Methods of generating protein molecules with these modifications are known in the art, e.g., as described in U.S. Pat. No. 5,731,168.

In certain embodiments, the Fc domain also comprises a triple mutation M252Y/S254T/T256E ("YTE"). This triple mutation has been reported to result in an approximately 10-fold increase in binding to the human neonatal Fc receptor (FcRn) and a nearly 4-fold increase in serum half-life of YTE-containing human IgG in cynomolgus monkeys (Oganesyan V. et al., Mol Immunol. 2009 May; 46 (8-9): 1750-5). In some further embodiments, the Fc domain comprises additional mutations to enhance the interaction between Fc and human FcRn.

In some embodiments, the Fc variant comprises or consists of an amino acid sequence that is at least 85%, at least 90%, or at least 99% identical to SEQ ID NO: 12.

### Characteristics of fusion proteins of the present disclosure

1. By fusion with Fc, the fusion protein's half-life is extended, the amount used of cytokines is reduced, and the toxic and side effect is decreased.
2. By structural modification of IL-2, the affinity of cytokines to IL-2Rβ and IL-2Rγ on the surface of NK cells and CD8+T cells is improved to enhance the killing effect of these cells on tumors.
3. The toxic and side effect of increased vascular permeability induced by IL-2 is significantly reduced by modification of amino acid at position 38 in IL-2.
4. The binding ability to the receptor IFNαR2 is reduced by modification of R144 and R149 to decrease the toxic and side effect while retaining the antitumor activity thereof.
5. Based on the above characteristics, the shortcomings of the current IL-2 and IFNα drugs are overcome by the fusion protein of structurally modified IL-2 with IFNα and Fc, which improves the therapeutic effect, decreases the toxic and side effect and expands the therapeutic safety window.

### Polynucleotides, vectors and host cells

In some aspects, the invention also provides polynucleotides encoding such fusion proteins. The polynucleotide can be used to express the fusion protein. In some embodiments, the polynucleotide can also be used as a therapeutic agent in an active form for achieving in vivo expression of a polypeptide fusion protein.

The polynucleotide encoding the fusion protein can be readily isolated and sequenced using conventional procedures known in the art. The polynucleotide can also be obtained by a synthetic method. Preferably, the polynucleotide is codon optimized for expression in eukaryotic host cells, in particular mammalian cells.

Polynucleotides encoding fusion proteins can be inserted into vectors for further cloning (amplification of DNA) or expression using known recombinant techniques. The components of vectors typically include, but are not limited to, one or more of the following: a signal sequence, an origin of replication, one or more marker genes, an enhancer element, a promoter (e.g., SV40, CMV, EF-1α) and a transcription termination sequence.

In some embodiments, the present disclosure provides a vector (e.g., an expression vector) comprising a polynucleotide encoding a fusion protein provided herein, at least one promoter (e.g., SV40, CMV, EF-1α) operably linked to the polynucleotide, and at least one selection marker. Examples of the vector include, but are not limited to, retroviruses (including lentiviruses), adenoviruses, adeno-associated viruses, herpesviruses (e.g., herpes simplex virus), poxviruses, baculoviruses, papillomaviruses, papovaviruses (e.g., SV40), λ bacteriophages and M13 bacteriophages, liposomes, plasmids pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM, pCDM8, pCDNA1.1 /amp, pcDNA3.1, pRc/RSV, PCR 2.1, pEF-1, pFB, pSG5, pXT1, pCDEF3, pSVSPORT, pEF-Bos, etc.

A vector comprising the polynucleotide sequence encoding the fusion protein can be introduced into a host cell for cloning or gene expression. Suitable host cells for cloning or expressing DNA in vectors herein are prokaryotes, yeasts, or higher eukaryote cells. Suitable prokaryotes for this purpose include eubacteria, such as Gram-negative or Gram-positive bacteria, for example, Escherichia coli. In addition to prokaryotes, eukaryotic microbes such as filamentous fungi or yeasts are suitable cloning or expression hosts for the provided vectors. *Saccharomyces cerevisiae,* or common baker's yeast, is the most commonly used among lower eukaryotic host microorganisms. However, a number of other genera, species, and strains are commonly available and useful herein.

Suitable host cells for the expression of fusion proteins provided herein can also be derived from multicellular organisms. Examples of invertebrate cells include plant and insect cells. In some preferred embodiments, the host cell is a Chinese hamster ovary (CHO) cell. In some other preferred embodiments, the host cells are other mammalian cell lines, such as human cell lines.

Host cells are transformed with the above-described expression or cloning vectors for producing fusion proteins, and cultured in conventional nutrient media modified as appropriate for inducing promoters, selecting transformants, or amplifying the genes encoding the desired sequences.

Host cells used to produce fusion proteins provided herein may be cultured in a variety of media. Any of these media may be supplemented as needed with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The fusion protein prepared from the cells can be purified using, for example, hydroxylapatite chromatography, gel electrophoresis, dialysis, DEAE-cellulose ion exchange chromatography, ammonium sulfate precipitation, salting out, and affinity chromatography, with the affinity chromatography being the preferred purification technique.

### Pharmaceutical compositions

In some aspects, the present disclosure provides a composition, such as a pharmaceutical composition, comprising the fusion protein herein formulated together with a pharmaceutically acceptable carrier. Such compositions comprise at least one fusion protein of the invention or a polynucleotide encoding the fusion protein.

As used herein, "pharmaceutically acceptable carriers" include any and all physiologically compatible and pharmaceutically acceptable liquid, gel or solid carriers, aqueous vehicles, non-aqueous vehicles, antimicrobial agents, isotonic agents, buffers, antioxidants, anesthetics, suspending/dispersing agents, sequestering or chelating agents, diluents, adjuvants, excipients or non-toxic auxiliary substances, other components known in the art or various combinations thereof, etc. The carrier can be suitable for intravenous, intramuscular, subcutaneous, parenteral, spinal, transepidermal, intravitreal injection or implant administration, and the like. Depending on the route of administration, the active compound, i.e., the ingredient of the present invention, may be coated in a material to protect the compound from acids and other natural conditions that may inactivate the compound.

The pharmaceutical composition of the present invention may further comprise a pharmaceutically acceptable antioxidant. Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials (such as lecithin), by the maintenance of the required particle size in the case of dispersing agents, and by the use of surfactants.

These compositions may also contain auxiliary agents such as preserving agents, wetting agents, emulsifying agents and dispersing agents. The pharmaceutical composition can be in the form of solid, paste, ointment, gel, liquid, aerosol, spray, polymer, film, emulsion or suspension.

In certain embodiments, the pharmaceutical composition is formulated into an injectable composition. The injectable pharmaceutical compositions may be prepared in any conventional form, such as liquid solution, suspension, emulsion, or solid forms suitable for generating a liquid solution, suspension, or emulsion. Preparations for injection may include sterile and/or non-pyrogenic solutions ready for injection, sterile dry soluble products, such as lyophilized powders, ready to be mixed with a solvent immediately prior to use, including hypodermic tablets, sterile suspensions ready for injection, sterile dry insoluble products ready to be combined with a vehicle immediately prior to use, and sterile and/or non-pyrogenic emulsions. The solutions may be either aqueous or non-aqueous. Pharmaceutically acceptable carriers include sterile aqueous solutions or dispersions and sterile powders for the extemporaneous preparation of sterile injectable solutions or dispersions.

The actual dosage levels of active ingredients and small molecules in the pharmaceutical composition of the present invention can be varied in order to obtain an amount of the active ingredient that is effective to achieve a desired therapeutic response for a particular patient, composition and mode of administration without being toxic to the patient. The selected dosage level depends on various pharmacokinetic factors, including the activity of the specific combination of the invention used, or esters, salts or amides thereof, route of administration, time of administration, excretion rate of the specific compound used, duration of treatment, other drugs, compounds and/or materials used in combination with the specific ingredient used, age, gender, weight, disorder, general health and past medical history of the patient to be treated, as well as similar factors well known in the medical field.

### Applications of the present disclosure

The fusion proteins, pharmaceutical compositions and methods of the present disclosure have numerous *in vitro* and *in vivo* utilities involving, for example, enhancement of immune response. For example, these molecules can be administered to cells in culture, in vitro or ex vivo, or to human subjects, e.g., in vivo, to enhance immunity in a variety of situations. The immune response can be modulated, for instance, augmented, stimulated or up-regulated.

For instance, the subjects include human patients in need of modulation of an immune response. In a particular embodiment, the methods are particularly suitable for treatment of cancer in vivo. To achieve enhancement of immunity, the fusion protein can be administered alone or in combination with another therapy. When the fusion protein is administered together with another agent, the two can be sequentially administered in any order or administered simultaneously.

In some aspects, the present disclosure provides a method of treating a disorder or a disease in a mammal, which comprises administering to the subject (for example, a human) in need of treatment a therapeutically effective amount of the fusion proteins as disclosed herein. The disorder or disease may be a cancer, such as a cancer treatable by IL-2 and IFNα. A variety of cancers, whether malignant or benign and whether primary or secondary, may be treated or prevented with a method provided by the disclosure. The cancers may be solid cancers or hematologic malignancies.

The fusion protein disclosed herein may be used alone as a monotherapy, or used in combination with cell immunotherapies, targeted therapies, chemotherapies or radiotherapies, and the like.

### Summary of sequences involved in the present invention

SEQ ID NO: 1 (Wild-type IL-2 amino acid sequence)
SEQ ID NO: 2 (IL-2 derivative, amino acid sequence) 
APTSSSTKKTQLQLEHLLLDLQMILNGINNYKNPKLTAMLTFKFYMPKKATELKHL
QCLEEELKPLEEVLNLAQSKNFHFDPRDVVSNINVFVLELKGSETTFMCEYADETATIVE
FLNR WITFCQSIISTLT
SEQ ID NO: 3 (IL-2-Fc fusion protein amino acid sequence) 
APTSSSTKKTQLQLEHLLLDLQMILNGINNYKNPKLTAMLTFKFYMPKKATELKHL
QCLEEELKPLEEVLNLAQSKNFHFDPRDVVSNINVFVLELKGSETTFMCEYADETATIVE
FLNRWITFCQSIISTLTGGGGSGGGGSGGGGSEPKSCDKTHTCPPCPAPEAAGGPSVFLFP
PKPKDTLYITREPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQG
NVFSCSVMHEALHNHYTQKSLSLSPK
SEQ ID NO: 4 (Fc-IL-2 fusion protein amino acid sequence) 
EPKSCDKTHTCPPCPAPEAAGGPSVFLFPPKPKDTLYITREPEVTCVVVDVSHEDPEV
KFNWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPENN
 
YKTTPPVLDSDGSFFLYSKLTVDKSRWQQGNVFSCSVMHEALHNHYTQKSLSLSPAGGG
GSGGGGSGGGGSAPTSSSTKKTQLQLEHLLLDLQMILNGINNYKNPKLTAMLTFKFYMP
KKATELKHLQCLEEELKPLEEVLNLAQSKNFHFDPRDVVSNINVFVLELKGSETTFMCEY
ADETATIVEFLNR WITFCQSIISTLT
SEQ ID NO: 5 (Wild-type IFNα amino acid sequence) 
CDLPQTHSLGSRRTLMLLAQMRRISLFSCLKDRHDFGFPQEEFGNQFQKAETIPVLH
EMIQQIFNLFSTKDSSAAWDETLLDKFYTELYQQLNDLEACVIQGVGVTETPLMNEDSIL
AVRKYFQRITLYLKEKKYSPCAWEVVRAEIMRSFSLSTNLQESLRSKE
SEQ ID NO: 6 (IFNα derivative, amino acid sequence) 
CDLPQTHSLGSRRTLMLLAQMRRISLFSCLKDRHDFGFPQEEFGNQFQKAETIPVLH
EMIQQIFNLFSTKDSSAAWDETLLDKFYTELYQQLNDLEACVIQGVGVTETPLMNEDSIL
AVRKYFQRITLYLKEKKYSPCAWEVVAAEIMRSFSLSTNLQESLRSKE
SEQ ID NO: 7 (IFNα-Fc fusion protein amino acid sequence) 
CDLPQTHSLGSRRTLMLLAQMRRISLFSCLKDRHDFGFPQEEFGNQFQKAETIPVLH
EMIQQIFNLFSTKDSSAAWDETLLDKFYTELYQQLNDLEACVIQGVGVTETPLMNEDSIL
AVRKYFQRITLYLKEKKYSPCAWEVVAAEIMRSFSLSTNLQESLRSKEGGGGSGGGGSG
GGGSEPKSCDKTHTCPPCPAPEAAGGPSVFLFPPKPKDTLYITREPEVTCVVVDVSHEDPE
VKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPK
SEQ ID NO: 8 (Fc-IFNα fusion protein amino acid sequence) 
EPKSCDKTHTCPPCPAPEAAGGPSVFLFPPKPKDTLYITREPEVTCVVVDVSHEDPEV
KFNWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPAGGG
GSGGGGSGGGGSCDLPQTHSLGSRRTLMLLAQMRRISLFSCLKDRHDFGFPQEEFGNQF
QKAETIPVLHEMIQQIFNLFSTKDSSAAWDETLLDKFYTELYQQLNDLEACVIQGVGVTE
TPLMNEDSILAVRKYFQRITLYLKEKKYSPCAWEVVAAEIMRSFSLSTNLQESLRSKE
SEQ ID NO: 9 (IL-2-Fc-IFNα fusion protein amino acid sequence) 
APTSSSTKKTQLQLEHLLLDLQMILNGINNYKNPKLTAMLTFKFYMPKKATELKHL
QCLEEELKPLEEVLNLAQSKNFHFDPRDVVSNINVFVLELKGSETTFMCEYADETATIVE
FLNRWITFCQSIISTLTGGGGSGGGGSGGGGSEPKSCDKTHTCPPCPAPEAAGGPSVFLFP
PKPKDTLYITREPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKN
 
QVSLTCLVKGFYPSDIAVEWESNGQPENNYKTTPPVLDSDGSFFLYSKLTVDKSRWQQG
NVFSCSVMHEALHNHYTQKSLSLSPAGGGGSGGGGSGGGGSCDLPQTHSLGSRRTLML
LAQMRRISLFSCLKDRHDFGFPQEEFGNQFQKAETIPVLHEMIQQIFNLFSTKDSSAAWD
ETLLDKFYTELYQQLNDLEACVIQGVGVTETPLMNEDSILAVRKYFQRITLYLKEKKYSP
CAWEVVAAEIMRSFSLSTNLQESLRSKE
SEQ ID NO: 10 (IFNα-Fc-IL-2 fusion protein amino acid sequence) 
CDLPQTHSLGSRRTLMLLAQMRRISLFSCLKDRHDFGFPQEEFGNQFQKAETIPVLH
EMIQQIFNLFSTKDSSAAWDETLLDKFYTELYQQLNDLEACVIQGVGVTETPLMNEDSIL
AVRKYFQRITLYLKEKKYSPCAWEVVAAEIMRSFSLSTNLQESLRSKEGGGGSGGGGSG
GGGSEPKSCDKTHTCPPCPAPEAAGGPSVFLFPPKPKDTLYITREPEVTCVVVDVSHEDPE
VKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPAGG
GGSGGGGSGGGGSAPTSSSTKKTQLQLEHLLLDLQMILNGINNYKNPKLTAMLTFKFYM
PKKATELKHLQCLEEELKPLEEVLNLAQSKNFHFDPRDVVSNINVFVLELKGSETTFMCE
YADETATIVEFLNR WITFCQSIISTLT
SEQ ID NO: 11 (Flexible linker amino acid sequence)
   GGGGSGGGGSGGGGS
SEQ ID NO: 12 (Fc moiety amino acid sequence)

EPKSCDKTHTCPPCPAPEAAGGPSVFLFPPKPKDTLYITREPEVTCVVVDVSHEDPEV
KFNWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPK

The present invention will be further elucidated below in conjunction with the accompanying drawings and specific implementation modes and it should be understood that the following specific implementation modes are merely used to illustrate the present invention and are not intended to limit the scope of the present invention.

### EXAMPLES

### Example 1: molecular construction and production of fusion proteins

An IL-2-Fc-IFNα fusion protein was constructed, comprising from N-terminal to C-terminal, an IL-2 moiety operably linked to an immunoglobulin Fc moiety and the immunoglobulin Fc moiety operably linked to an IFNα moiety, which was hereinafter referred to as IL-2-Fc-IFNα and the amino acid sequence of which was as set forth in SEQ ID NO: 9. An IL-2-Fc fusion protein was also constructed, comprising from N-terminal to C-terminal, an IL-2 moiety operably linked to an immunoglobulin Fc moiety, which was hereinafter referred to as IL-2-Fc and the amino acid sequence of which was as set forth in SEQ ID NO: 3. Furthermore, an Fc-IFNα fusion protein was constructed, comprising from N-terminal to C-terminal, an immunoglobulin Fc moiety operably linked to an IFNα moiety, which was hereinafter referred to as Fc-IFNα and the amino acid sequence of which was as set forth in SEQ ID NO: 8.

The sequences were amplified by PCR using conventional molecular biological techniques, and the genes encoding amino acid sequences were ligated into the pcDNA3.4 vector by the homologous recombination technology. Plasmids were extracted from the sequenced positive clones and transfected into Expi293F cells, which were continued to be cultured in a shaker at 37°C/5%CO₂/125 rpm for 7 days, and at the end of the culture, the supernatant was collected and subjected to protein A affinity chromatography to obtain purified fusion protein molecules. Protein concentration was determined by UV280 combined with the theoretical extinction coefficient.

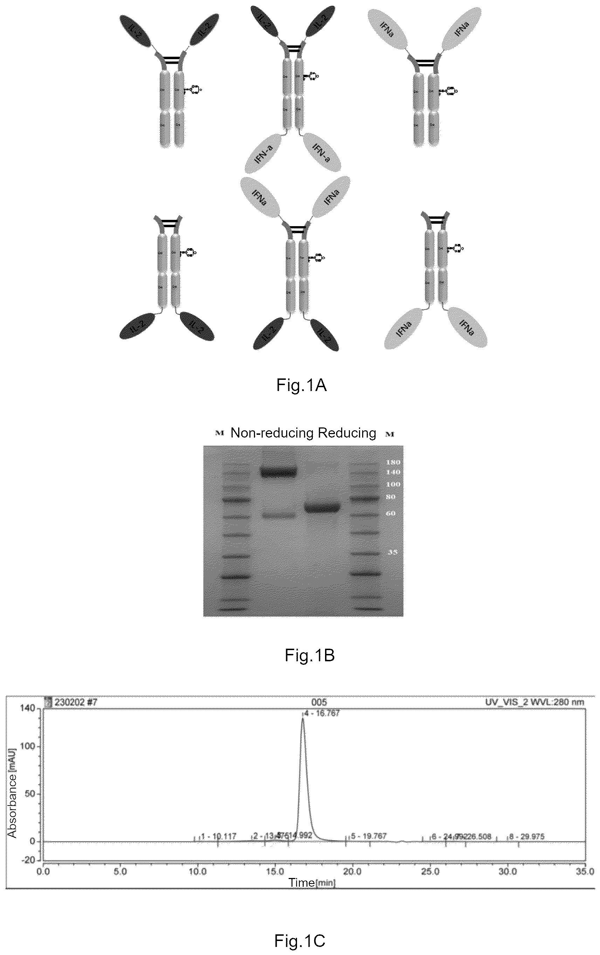

Figure 1B showed an SDS PAGE gel image of the IL-2-Fc-IFNα fusion protein, and Figure 1C showed its HPLC purity analysis graph.

### Example 2: Binding ability to receptor IL-2Rα protein by ELISA

The IL-2Rα protein (10165-H08H, Sino Biological) was coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IFNα2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

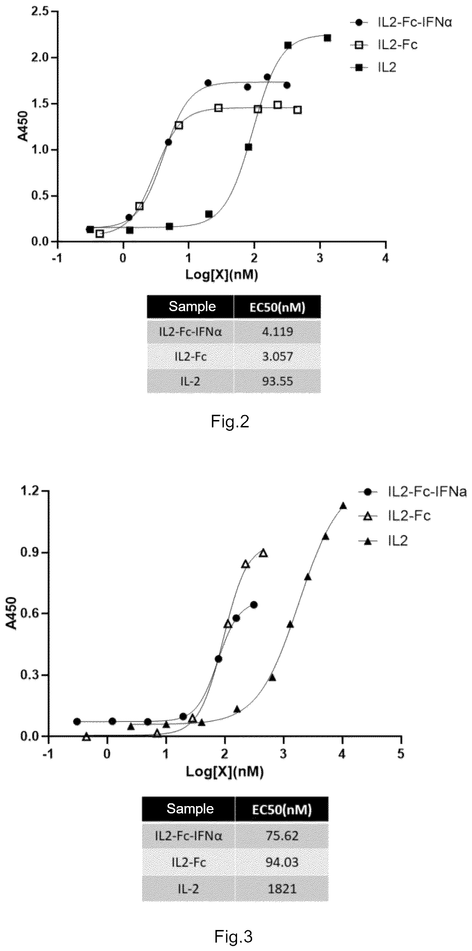

The results were shown in Figure 2. The binding capacity of IL-2-Fc and IL-2-Fc-IFNα to the receptor IL-2Rα protein was significantly improved compared with the wild-type IL-2 (11848-HNAH1-E, Sino Biological).

### Example 3: Binding ability to receptor IL-2Rβ protein by ELISA

The IL-2Rβ protein (10696-H05H, Sino Biological) was coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IFNα2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

The results were shown in Figure 3. The binding capacity of IL-2-Fc and IL-2-Fc-IFNα to the receptor IL-2Rβ protein was improved by more than 24 times compared with the wild-type IL-2.

### Example 4: Binding ability to receptor IFNαR2 protein by ELISA

The IFNαR2 protein (10359-H02H, Sino Biological) was coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IL-2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

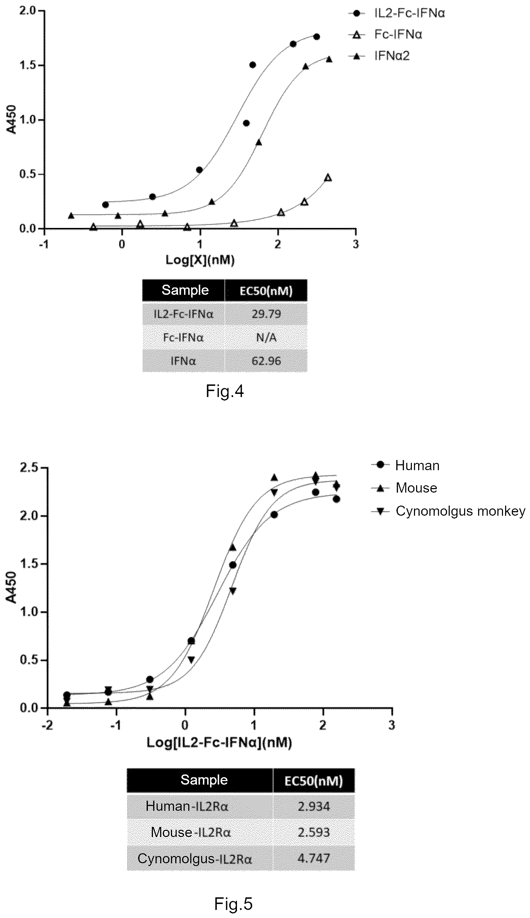

The results were shown in Figure 4. Compared with the wild-type IFNα, the binding ability of Fc-IFNα to the receptor IFNαR2 protein was significantly reduced, while the binding ability of IL-2-Fc-IFNα to the receptor IFNαR2 protein was significantly increased.

### Example 5: Binding ability to receptor IL-2Rα proteins across different species by ELISA

Human, mouse and cynomolgus monkey IL-2Rα proteins were separately coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IFNα2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

The results were shown in Figure 5. It was confirmed that IL-2-Fc-IFNα had a similar binding capacity to human, mouse, and cynomolgus monkey receptor IL-2Rα proteins, indicating a cross-species binding in mice and cynomolgus monkeys.

### Example 6: Binding ability to receptor IL-2Rβ proteins across different species by ELISA

Human, mouse and cynomolgus monkey IL-2Rβ proteins were separately coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IFNα2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

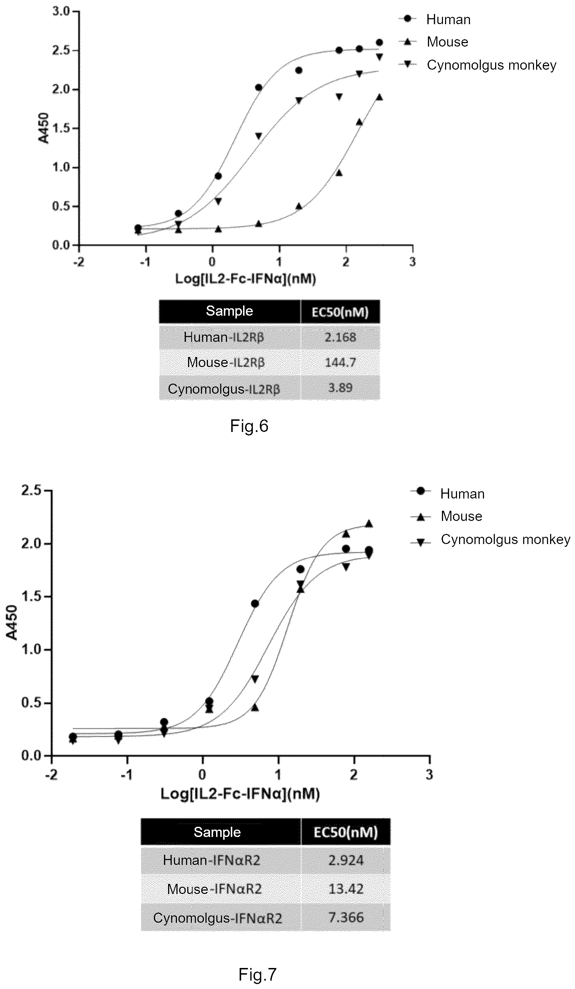

The results were shown in Figure 6. Experiments confirmed that IL-2-Fc-IFNα had a cross-species binding to human, mouse, and cynomolgus monkey receptor IL-2Rβ proteins. The binding ability to the cynomolgus monkey IL-2Rβ protein was similar to that to the human IL-2Rβ protein, and the binding to the mouse IL-2Rβ protein was lower than that to the human IL-2Rβ protein.

### Example 7: Binding ability to receptor IFNαR2 proteins across different species by ELISA

Human, mouse and cynomolgus monkey IFNαR2 proteins were separately coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IL-2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

The results were shown in Figure 7. It was confirmed that IL-2-Fc-IFNα bound to human, mouse, and cynomolgus monkey receptor IFNαR2 proteins, demonstrating a cross-species binding.

### Example 8: Detection of binding ability to FcRn protein

### 8.1 Binding ability to FcRn protein by ELISA

The FcRn protein (10359-H02H, Sino Biological) was coated at a concentration of 2 µg/ml, 100 µl/well, and kept overnight at 4°C; 300 µl/well of 3% skimmed milk was used for blocking at 37°C for 1 h; each well was added with 100 µl of different concentrations of fusion proteins and their control samples, and incubated at 37°C for 1 h; anti-IFNα2 Rabbit pAb was then added and the mixture was incubated at 37°C for 1 h, followed by addition of anti-Rabbit IgG HRP and continued incubation for 1 h at 37°C; each well was added with 100 µl of TMB for 5 min of color development before addition of 50 µl of termination solution, and OD450 was read on a microplate reader.

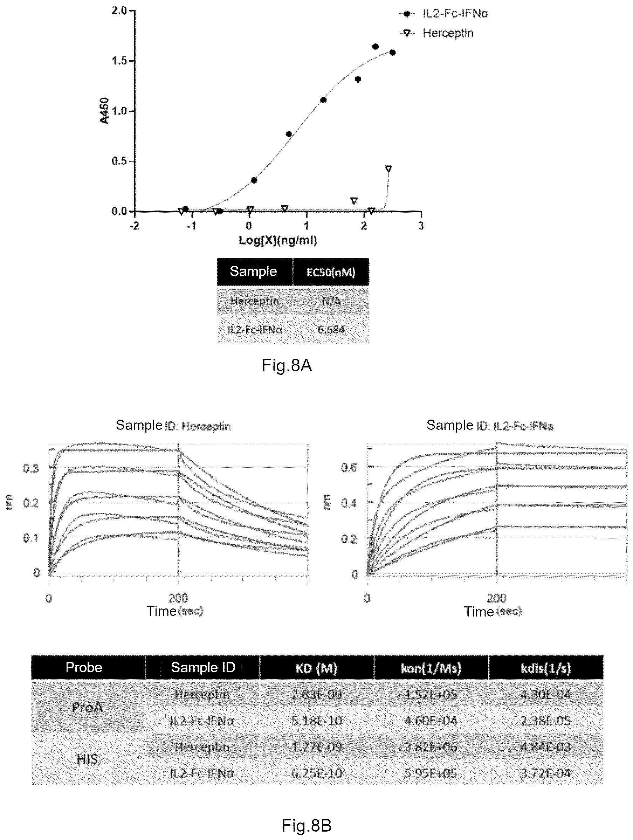

The results were shown in Figure 8A. Experiments confirmed that IL-2-Fc-IFNα had a significantly increased binding affinity compared to the Herceptin antibody (Trastuzumab).

### 8.2 Fortebio detection of binding ability to FcRn protein

The proteins to be tested were loaded with ProA and HIS probes at a concentration of 4 µg/ml, and then bound to and dissociated from different concentrations of FcRn (4 µg/ml, 1 µg/ml, 0.25 µg/ml, and 0), and the affinity between the molecules to be tested and FcRn was calculated.

The results were shown in Figure 8B. Experiments confirmed that IL-2-Fc-IFNα had a significantly increased affinity for FcRn protein compared to the Herceptin antibody (Trastuzumab).

### Example 9: Detection of immune cell subsets after stimulation of human PBMC cells

Fresh human PBMCs were plated into a 96-well plate at 150,000 cells/well, and 500 nM proteins were added according to the experimental design followed by continued culture for 72 h. At the end of incubation, staining was performed as required for flow cytometry detection to label Anti-Foxp3 Antibody (FITC), Anti-CD4 Antibody (PE), Anti-CD56 Antibody (PE), Anti-CD8 Antibody (PE), and Anti-Granzyme B Antibody (FITC), respectively, and the flow cytometry data were collected and analyzed for data summarization and analysis.

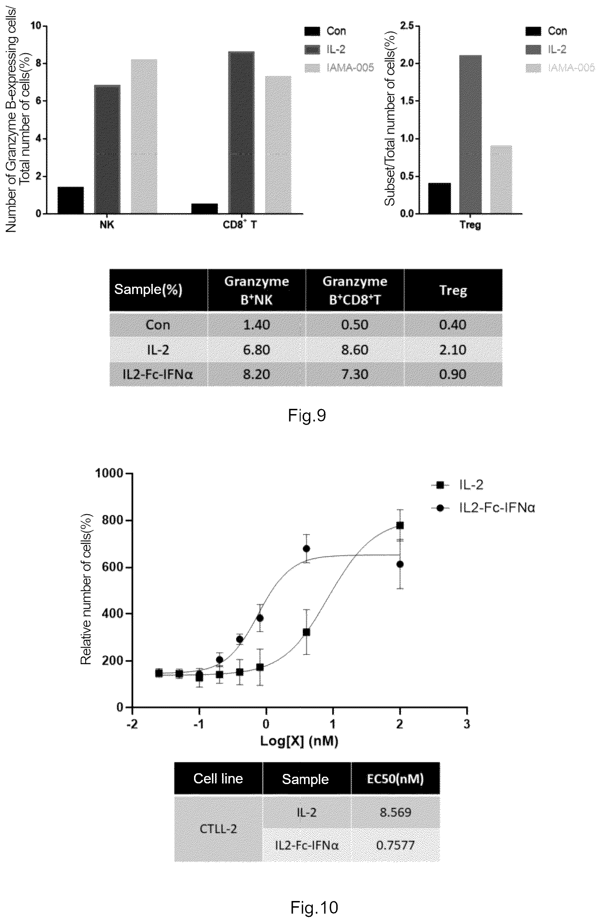

The results were shown in Figure 9. The experiment confirmed that the ability of IL-2-Fc-IFNα to stimulate the activation of NK and CD8+T cells in PBMCs and expression of Granzyme B maintained the same activity as that of the wild-type IL-2. The ability to stimulate the proliferation of Treg cells (FOXP3⁺ cells) was significantly reduced compared to the wild-type IL-2.

### Example 10: Detection of the ability to stimulate proliferation of CTLL-2 mouse T lymphocytes

After cultured and expanded to the required cell number, the mouse CTLL-2 cells were plated into a 96-well plate at 100,000 cells/well, and different concentrations (100 nM, 4 nM, 800 pM, 400 pM, 200 pM, 100 pM, 50 pM, 25 pM, 0) of proteins were added according to the experimental design, followed by continued culture for 72 h. The CCK-8 method was used to determine the expression amount of Formazan in live cells, and then the cell viability was evaluated. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

The results were shown in Figure 10. The experiment confirmed that the ability of IL-2-Fc-IFNα to stimulate the proliferation of CTLL-2 cells was 10 times higher than that of the wild-type IL-2.

### Example 11: Detection of direct killing ability against tumor cells in vitro

Direct killing ability refers to a determination of the direct killing against tumor cells by IFN-a in the absence of immune cells.

### 11.1 Detection of direct killing ability against human gastric cancer cell line NCI-N87

After cultured and expanded to the required cell number, NCI-N87 human gastric cancer cells were plated into a 96-well plate at 4,000 cells/well, and different concentrations (4000 nM, 1000 nM, 250 nM, 100 nM, 62.5 nM, 15.625 nM, 10 nM, 4 nM, 1.5625 nM, 1 nM, 244.14 pM, 156.25 pM, 100 pM, 100 pM, 10 pM, 1pM, 0) of proteins were added according to the experimental design, followed by continued culture for 5 days. The CCK-8 method was used to determine the expression amount of Formazan in live cells, and then the cell viability was evaluated. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

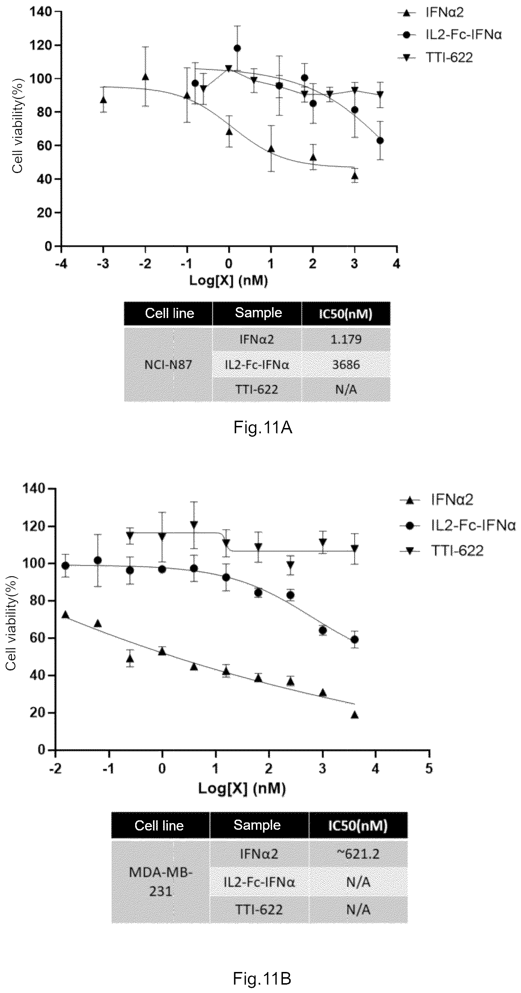

The results were shown in Figure 11A. Wild-type IFNα had a significant killing effect against NCI-N87 cells; however, IL-2-Fc-IFNα had a killing effect on NCI-N87 cells only at high concentrations. The negative control, TTI-622 (SIRPa-Fc), had no killing function against NCI-87 cells.

### 11.2 Detection of direct killing ability against human breast cancer cell line MDA-MB-231

After cultured and expanded to the required cell number, MDA-MB-231 human breast cancer cells were plated into a 96-well plate at 2,000 cells/well, and different concentrations (4000 nM, 1000 nM, 250 nM, 62.5 nM, 15.625 nM, 4 nM, 1 nM, 244.14 pM, 61 pM, 15.26 pM, 0) of proteins were added according to the experimental design, followed by continued culture for 5 days. The CCK-8 method was used to determine the expression amount of Formazan in live cells, and then the cell viability was evaluated. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

The results were shown in Figure 11B. Wild-type IFNα had a significant killing function against MDA-MB-231 cells; however, IL-2-Fc-IFNα had a killing effect on MDA-MB-231 cells only at high concentrations. The negative control, TTI-622 (SIRPa-Fc), had no killing function against MDA-MB-231 cells.

### 11.3 Detection of direct killing ability against human melanoma cell line A375

After cultured and expanded to the required cell number, A375 human melanoma cells were plated into a 96-well plate at 1,000 cells/well, and different concentrations (16000 nM, 1600 nM, 160 nM, 16 nM, 1.6 nM, 160 pM, 16 pM, 1.6 pM, 0.16 pM, 0) of proteins were added according to the experimental design, followed by continued culture for 5 days. The CCK-8 method was used to determine the expression amount of Formazan in live cells, and then the cell viability was evaluated. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

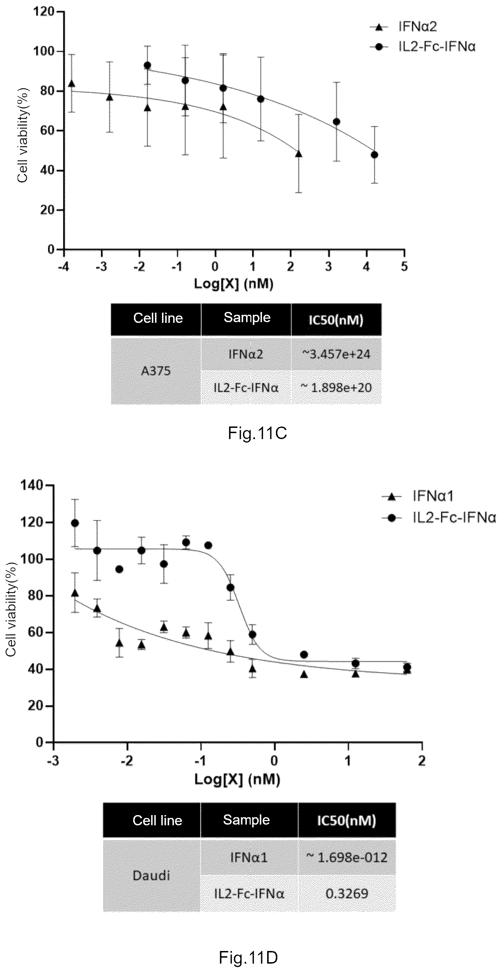

The results were shown in Figure 11C. Wild-type IFNα had a significant killing function against A375 cells; however, IL-2-Fc-IFNα had a killing effect on A375 cells only at high concentrations. The negative control, TTI-622 (SIRPa-Fc), had no killing function against A375 cells.

### 11.4 Detection of direct killing ability against human Burkitt's lymphoma cell line Daudi B

After cultured and expanded to the required cell number, Daudi B human Burkitt's lymphoma cells were plated into a 96-well plate at 10,000 cells/well, and different concentrations (62.5 nM, 12.5 nM, 2.5 nM, 500 pM, 250 pM, 125 pM, 62.5 pM, 31.25 pM, 15.625 pM, 7.813 pM, 4 pM, 2 pM, 0) of proteins were added according to the experimental design, followed by continued culture for 5 days. The CCK-8 method was used to determine the expression amount of Formazan in live cells, and then the cell viability was evaluated. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

The results were shown in Figure 11D. Wild-type IFNα had a significant killing function against Daudi B cells; however, IL-2-Fc-IFNα had a killing effect on Daudi B cells only at higher concentrations. The negative control, TTI-622 (SIRPa-Fc), had no killing function against Daudi B cells.

The experiment confirmed that the direct killing function of the fusion protein herein was weaker than that of wild-type IFNα2, but it still retained the direct killing function of the IFNα arm.

### Example 12: Detection of comprehensive killing ability against tumor cells in vitro

Comprehensive killing function refers to a sum of direct killing capability and indirect killing capability. The comprehensive killing ability is obtained in the presence of immune cells like PBMCs.

### 12.1 Detection of comprehensive killing ability against human gastric cancer cell line NCI-N87

After cultured and expanded to the required cell number, NCI-N87 human gastric cancer cells were plated into a 96-well plate at 4,000 cells/well and allowed to adhere for 6 hours. After fresh human PBMCs were procured, the cells were plated according to an effector-target ratio of 10:1, and different concentrations (800 nM, 80 nM, 8 nM, 800 pM, 80 pM, 8 pM, 0.8 pM, 0) of proteins were added according to the experimental design, followed by continued culture for 5 days. The plates were washed three times with PBS to remove PBMCs, and the CCK-8 method was used to determine the expression amount of Formazan in live cells to evaluate cell viability. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

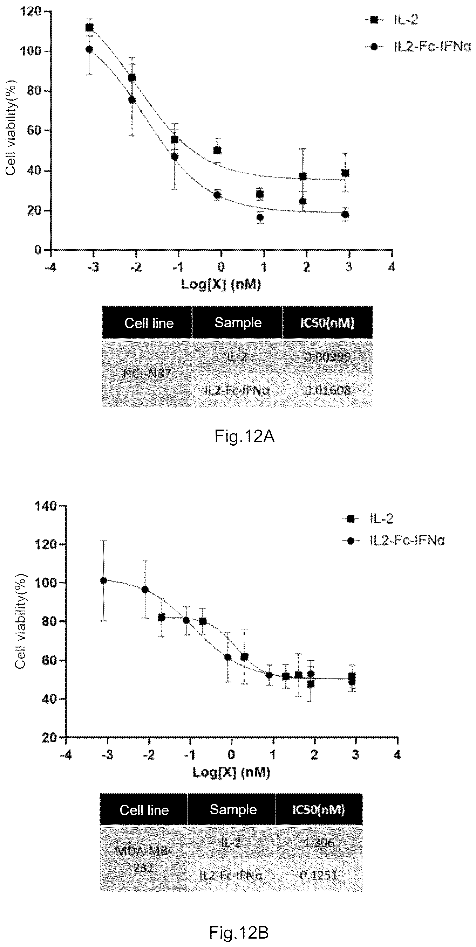

The results were shown in Figure 12A. The comprehensive killing ability of IL-2-Fc-IFNα against NCI-N87 cells was stronger than that of wild-type IL-2.

### 12.2 Detection of comprehensive killing ability against human breast cancer cell line MDA-MB-231

After cultured and expanded to the required cell number, MDA-MB-231 human breast cancer cells were plated into a 96-well plate at 4,000 cells/well and allowed to adhere for 6 hours. After fresh human PBMCs were procured, the cells were plated according to an effector-target ratio of 10:1, and different concentrations (800 nM, 80 nM, 8 nM, 800 pM, 80 pM, 8 pM, 0.8 pM, 0) of proteins were added according to the experimental design, followed by continued culture for 5 days. The plates were washed three times with PBS to remove PBMCs, and the CCK-8 method was used to determine the expression amount of Formazan in live cells to evaluate cell viability. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

The results were shown in Figure 12B. The comprehensive killing ability of IL-2-Fc-IFN-a against MDA-MB-231 cells was similar to that of wild-type IL-2.

### 12.3 Detection of comprehensive killing ability against human melanoma cell line A375

After cultured and expanded to the required cell number, A375 human melanoma cells were plated into a 96-well plate at 4,000 cells/well and allowed to adhere for 6 hours. After fresh human PBMCs were procured, the cells were plated according to an effector-target ratio of 10:1, and different concentrations (800 nM, 80 nM, 8 nM, 800 pM, 80 pM, 8 pM, 0.8 pM, 0) of proteins were added according to the experimental design, followed by continued culture for 5 days. The plates were washed three times with PBS to remove PBMCs, and the CCK-8 method was used to determine the expression amount of Formazan in live cells to evaluate cell viability. The specific operations were carried out according to the instructions, in brief, 10% CCK-8 solution was added after the culture was completed, well mixed and then incubated for 2-4 hours. The absorbance value data were collected in a microplate reader. The data were analyzed and processed by Excel, and the GraphPad Prism 7 software was used to fit the pharmacodynamic dose curve based on the absorbance value data to calculate the IC50.

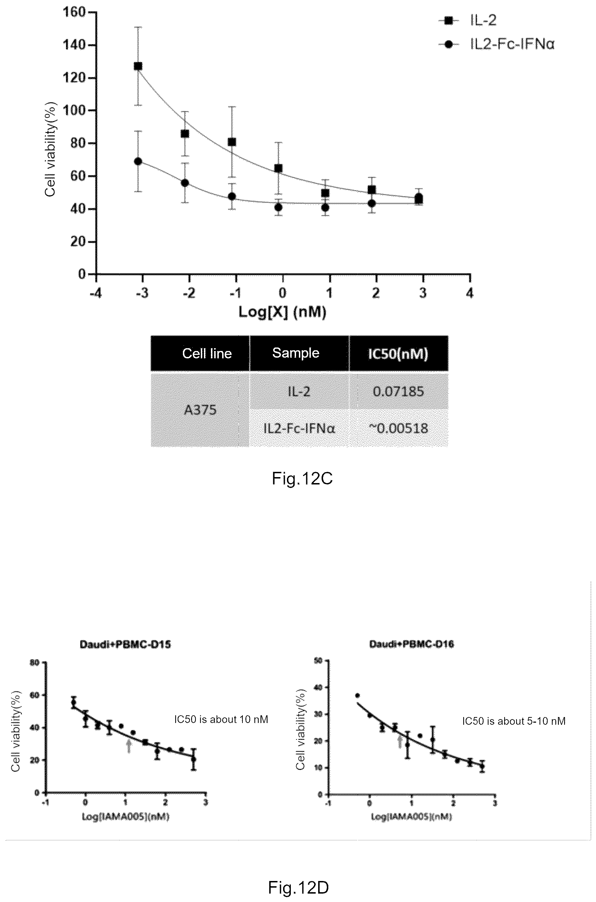

The results were shown in Figure 12C. The comprehensive killing ability of IL-2-Fc-IFN-a against A375 cells was significantly stronger than that of wild-type IL-2.

### 12.4 Detection of comprehensive killing ability against human Burkitt's lymphoma cell line Daudi B

Daudi B human Burkitt's lymphoma cells were cultured and expanded to the required cell number. Fresh PBMCs were collected and isolated from the donor. Daudi B cells were plated into 96-well plates at 10,000 cells/well, with two replicate wells, and PBMCs (with the effector-target ratios set to 10:1 and 5:1) and different concentrations of IAMA005 (i.e. IL-2-Fc-IFN-a, at 16 nM or 160 nM) were added into the corresponding well plates according to the experimental layout design, with a total of 3 plates, which were taken and used separately according to different detection times. After Co-culturing for 24 h, 48 h, and 72 h, the culture supernatant was sequentially collected and retained into the LDH detection preservation solution, and stored in a -80°C freezer. The cells in each well were resuspended separately, followed by staining and detection in accordance with the requirements for flow cytometry, and the flow cytometry data were collected and analyzed for data summarization and analysis. The results were shown in Figure 12D.

After cultured and expanded to the required cell number, Daudi B human Burkitt's lymphoma cells were stained with CFSE, and then the Daudi B cells were plated into 96-well plates at 20,000 cells/well. After fresh human PBMCs were procured, the cells were plated according to an effector-target ratio of 10:1, and different concentrations (800 nM, 80 nM, 8 nM, 800 pM, 80 pM, 8 pM, 0.8 pM, 0) of proteins were added according to the experimental design, followed by continued culture for 5 days. The plates were washed three times with PBS and centrifuged at low speed to remove cell debris, and a fluorescent microplate reader was adopted to determine the fluorescence intensity in live cells to evaluate cell viability. The Excel software was used to analyze and process the data, and the GraphPad Prism 7 software was used to fit the pharmacodynamic dose curve based on the absorbance value data to calculate the IC50.

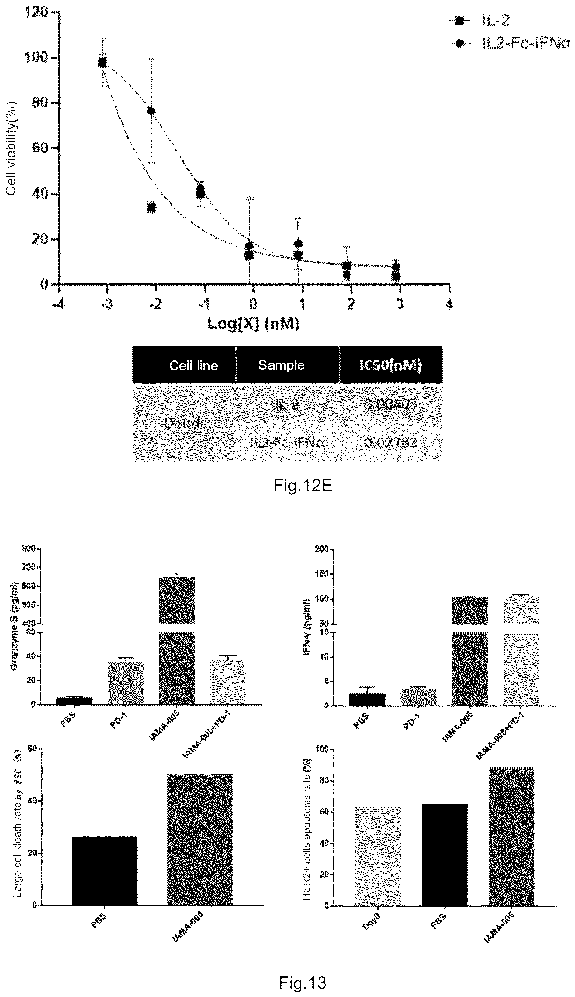

The results were shown in Figure 12E. The comprehensive killing ability of IL-2-Fc-IFN-a against Daudi B cells maintained an activity comparable to that of wild-type IL-2.

### Example 13: Pharmacodynamics study in an ExVivo organoid system

A pleural fluid sample (identified as positive HER2 2+) from a patient with breast cancer was collected, and cells in the sample, including tumor cells and immune cells, were isolated. Candidate drugs and reference drugs were co-incubated with tumor cell-immune cell complexes in vitro. After 4-5 days of treatment, the culture media and cells were collected for analysis, including tumor cells, Granzyme B, IFN-γ, etc.

The results were shown in Figure 13. In the ExVivo evaluation system of this sample, the antitumor activity of pleural fluid immune cells stimulated by IL-2-Fc-IFN-a was significantly elevated, and their secreted antitumor cytokines Granzyme B and IFN-γ were also significantly increased.

Exemplary examples of the present disclosure are described above in conjunction with the accompanying drawings. However, it should be understood by those skilled in the art that the present disclosure is not limited to the specific structures disclosed. Various changes and modifications can be made to the exemplary examples of the disclosure without departing from the spirit and scope of the disclosure. All these changes and modifications are included within the protection scope defined by the claims of the present disclosure.

### REFERENCES

[1] Yang Y, Lundqvist A. Immunomodulatory effects of IL-2 and IL-15; implications for cancer immunotherapy[J]. Cancers, 2020, 12(12): 3586.
[2] Choudhry H, Helmi N, Abdulaal W H, et al. Prospects of IL-2 in cancer immunotherapy[J]. BioMed research international, 2018, 2018.
[3] Levin A M, Bates D L, Ring A M, et al. Exploiting a natural conformational switch to engineer an interleukin-2 'superkine'[J]. Nature, 2012, 484(7395): 529-533.
[4] Khailaie S, Montaseri G, Meyer-Hermann M. An adaptive control scheme for Interleukin-2 therapy[J]. Iscience, 2020, 23(11): 101663.
[5] Cauwels A, Van Lint S, Garcin G, et al. A safe and highly efficient tumor-targeted type I interferon immunotherapy depends on the tumor microenvironment[J]. Oncoimmunology, 2018, 7(3): e1398876.
[6] Garcin G, Paul F, Staufenbiel M, et al. High efficiency cell-specific targeting of cytokine activity[J]. Nature communications, 2014, 5(1): 1-9.
[7] Piehler J, Roisman L C, Schreiber G. New structural and functional aspects of the type I interferon-receptor interaction revealed by comprehensive mutational analysis of the binding interface[J]. Journal of biological chemistry, 2000, 275(51): 40425-40433.
[8] Sim G C, Liu C, Wang E, et al. IL2 variant circumvents ICOS+ regulatory T-cell expansion and promotes NK cell activation[J]. Cancer Immunology Research, 2016, 4(11): 983-994.
[9] Heaton K M, Ju G, Morris D K, et al. Characterization of lymphokine-activated killing by human peripheral blood mononuclear cells stimulated with interleukin 2 (IL-2) analogs specific for the intermediate affinity IL-2 receptor[J]. Cellular immunology, 1993, 147(1): 167-179.
[10] Fukushima K, Yamashita K. Interleukin-2 carbohydrate recognition modulates CTLL-2 cell proliferation[J]. Journal of Biological Chemistry, 2001, 276(10): 7351-7356.
[11] Luo F, Qian J, Yang J, et al. Bifunctional αHER2/CD3 RNA-engineered CART-like human T cells specifically eliminate HER2+ gastric cancer[J]. Cell Research, 2016, 26(7): 850-853.
[12] Chen X, Ai X, Wu C, et al. A novel human IL-2 mutein with minimal systemic toxicity exerts greater antitumor efficacy than wild-type IL-2[J]. Cell death & disease, 2018, 9(10): 989.
[13] Carmenate T, Pacios A, Enamorado M, et al. Human IL-2 mutein with higher antitumor efficacy than wild type IL-2[J]. The Journal of Immunology, 2013, 190(12): 6230-6238.

## Claims

1. A fusion protein comprising an IL-2 moiety and an Fc moiety, wherein the IL-2 moiety comprises an amino acid sequence with one or more mutations compared to wild-type IL-2 protein, and the amino acid sequence has with at least 90% identity to SEQ ID NO: 2.

2. The fusion protein of claim 1, wherein the mutations include one or more substitutions selected from the following with reference to amino acid positions in SEQ ID NO: 1: R38A, L80F, R81D, L85V, I86V and I91F.

3. The fusion protein of claim 1 or 2, wherein the Fc moiety comprises a human IgG Fc, such as human IgG1 Fc, IgG2 Fc, IgG3 Fc, IgG4 Fc or variants thereof.

4. The fusion protein of claim 3, wherein the Fc variant comprises one or more mutations selected from the following: L234A and L235A mutations, and M252Y, S254T and T256E mutations.

5. The fusion protein of any one of claims 1-4, wherein the fusion protein further comprises an IFNα moiety.

6. The fusion protein of claim 5, wherein the IFNα moiety comprises an amino acid sequence with at least 90% identity to SEQ ID NO: 6 or 5.

7. The fusion protein of claim 6, wherein the IFNα moiety includes one or more substitutions selected from the following with reference to amino acid positions in SEQ ID NO: 5: R144A and R149A.

8. The fusion protein of any one of claims 1-7, comprising:
(a) the IL-2 moiety operably linked to the Fc moiety;
(b) the IL-2 moiety operably linked to the Fc moiety, and the Fc moiety operably linked to the IFNα moiety;
(c) the IL-2 moiety operably linked to the IFNα moiety, and the IFNα moiety operably linked to the Fc moiety; or
(d) the IFNα moiety operably linked to the IL-2 moiety, and the IL-2 moiety operably linked to the Fc moiety.

9. The fusion protein of claim 8, comprising from N-terminal to C-terminal:
(a) the IL-2 moiety operably linked to the Fc moiety;
(b) the IL-2 moiety operably linked to the Fc moiety, and the Fc moiety operably linked to the IFNα moiety;
(c) the Fc moiety operably linked to the IL-2 moiety; or
(d) the IFNα moiety operably linked to the Fc moiety, and the Fc moiety operably linked to the IL-2 moiety.

10. The fusion protein of claim 8 or 9, wherein operable linked is a direct linkage or a linking via a peptide linker, and optionally the peptide linker is a GS series linker, such as the peptide linker as set forth in SEQ ID NO: 11.

11. The fusion protein of any one of claims 1-10, comprising the amino acid sequence as set forth in SEQ ID NO: 3, 4, 9 or 10.

12. A fusion protein comprising an IFNα moiety and an Fc moiety, wherein the IFNα moiety comprises an amino acid sequence with one or more mutations compared to wild-type IFNα protein, and the amino acid sequence has at least 90% identity to SEQ ID NO: 6.

13. The fusion protein of claim 12, wherein the IFNα moiety includes one or more substitutions selected from the following with reference to amino acid positions in SEQ ID NO: 5: R144A and R149A.

14. The fusion protein of claim 12 or 13, wherein the Fc moiety comprises a human IgG Fc, such as human IgG1 Fc, IgG2 Fc, IgG3 Fc, IgG4 Fc or variants thereof.

15. The fusion protein of claim 14, wherein the Fc variant comprises one or more mutations selected from the following: L234A and L235A mutations, and M252Y, S254T and T256E mutations.

16. The fusion protein of any one of claims 12-15, comprising from N-terminal to C-terminal:
(a) the IFNα moiety operably linked to the Fc moiety; or
(b) the Fc moiety operably linked to the IFNα moiety.

17. The fusion protein of claim 16, comprising the amino acid sequence as set forth in SEQ ID NO: 7 or 8.

18. A nucleic acid molecule comprising a nucleic acid sequence encoding the fusion protein of any one of claims 1-16.

19. A vector comprising the nucleic acid molecule of claim 18.

20. A host cell comprising the nucleic acid molecule of claim 18 or the vector of claim 19.

21. A pharmaceutical composition comprising the fusion protein of any one of claims 1-16 or the nucleic acid molecule encoding the same, and a pharmaceutically acceptable carrier.

22. A method for producing the fusion protein of any one of claims 1-16, comprising the following steps:
- expressing the fusion protein in a host cell comprising a vector encoding the fusion protein; and
- isolating the fusion protein from the host cell culture.

23. A method of modulating an immune response in a subject, comprising administering to the subject the fusion protein of any one of claims 1-16 or the pharmaceutical composition of claim 21.

24. A method for treating or preventing a cancer or an infectious disease in a subject, comprising administering to the subject an effective amount of the fusion protein of any one of claims 1-16 or the pharmaceutical composition of claim 21.

25. The method of claim 24, wherein the cancer is selected from breast cancer, gastric cancer, melanoma, lymphoma, lung cancer, colon cancer, ovarian cancer, bladder cancer, renal cell carcinoma, liver cancer, prostate cancer, pancreatic cancer and leukemia.

26. Use of the fusion protein of any one of claims 1-16 in the manufacture of a medicament for the prevention, treatment and/or management of a cancer or an infectious disease.

27. The fusion protein of any one of claims 1-16 for use in the treatment or prevention of a cancer or an infectious disease.

28. A kit comprising a container containing the fusion protein of any one of claims 1-16 or the pharmaceutical composition of claim 21.
